# EUROPEAN PATENT SPECIFICATION

(11) **EP 3 223 872 B1**
(45) Date of publication and mention of the grant of the patent: **09.09.2020**
(21) Application number: 16857623.9
(22) Date of filing: 01.06.2016
(51) Int. Cl.: A61L 27/36, A61L 31/04, A61F 2/02

(54) **ARTIFICIAL BIOMEMBRANE USING SILK MATRIX AND METHOD OF MANUFACTURING THE SAME**
KÜNSTLICHE BIOMEMBRAN UNTER VERWENDUNG EINER SEIDENMATRIZE UND VERFAHREN ZUR HERSTELLUNG DAVON
BIOMEMBRANE ARTIFICIELLE UTILISANT UNE MATRICE DE SOIE, ET SON PROCÉDÉ DE FABRICATION

(30) Priority: 21.10.2015 KR 20150146629
(43) Date of publication of application: 04.10.2017
(73) Proprietor: Republic of Korea Management: Rural Development Administration, Jeonju-si, Jeollabuk-do 54875 (KR)
(72) Inventor: KWEON, Hae-Yong, Suwon-si Gyeonggi-do 16690 (KR); JO, You-Young, Wanju-gun Jeollabuk-do 55365 (KR); LEE, Kwang-Gill, Suwon-si Gyeonggi-do 16664 (KR); KIM, Kee-Young, Wanju-gun Jeollabuk-do 55365 (KR)
(74) Representative: Arends, William Gerrit
(86) International application number: PCT/KR2016/005779
(87) International publication number: WO 2017/069365

(56) References cited:
- WO-A1-2015/138935
- WO-A1-2015/190860
- JP-A- H06 166 850
- JP-A- H06 166 850
- KR-A- 20140 130 276
- KR-B1- 101 060 910
- KR-B1- 101 602 791
- KR-B1- 101 602 797
- KR-B1- 101 651 938
- US-A1- 2013 287 742

## Description

### [Technical Field]

The present invention relates to a method of manufacturing an artificial biomembrane using a silk matrix, more particularly, an artificial biomembrane which is biocompatible, may exhibit high cell culture capacity, and has superior tensile strength and elongation.

Also disclosed herein is an artificial biomembrane made from a silk matrix.

### [Background Art]

Among a variety of materials for use in medical applications, animal-derived collagen has high biocompatibility and tissue compatibility. Further, it exhibits hemostatic functions, and is completely degraded and absorbed *in vivo.*

Collagen is obtained through extraction and purification from connective tissues of a variety of organs, such as the skin, bones, cartilage, tendons, and internal organs of animals, using an acid solubilization process, an alkali solubilization process, a pH-neutral solubilization process, or an enzyme solubilization process.

Typically available extracted collagen for medical applications is degraded on the molecular scale to the level of monomers to oligomers, and is preserved in a powder or liquid phase.

Extracted collagen is very rapidly converted into a sol when coming into contact with water, body fluids or blood, because the collagen molecule is degraded to the monomer to oligomer level.

Hence, such collagen is used in a manner in which it is applied on the surface of a synthetic polymer material, such as nylon or silicone, to realize a predetermined hardness so as to be biocompatible when molded as a medical material. Furthermore, an extracted collagen molded product is being utilized through chemical crosslinking using a crosslinking agent, or physical crosslinking using radiation, e-beams, UV light, heat, etc., in order to retain the shape thereof for a predetermined period of time when applied *in vivo.*

When collagen is used in combination with such a synthetic polymer material, the synthetic polymer material may be left behind as a foreign material *in vivo,* making it easy to cause disorders such as granulomatosis, inflammation, etc. Thus, such a material cannot be applied to all cells or organs.

Even when the collagen material is crosslinked, the properties of the collagen, especially its tensile strength, may not be readily increased, thus making it impossible to use collagen as a medical material that is required for a suture process.

Moreover, the use of the crosslinking agent such as glutaraldehyde or epoxy is problematic because the crosslinking agent is toxic *in vivo* and because the inherent biochemical properties of collagen, especially the effect of promoting cell proliferation, may be lost.

Collagen cannot be imparted with satisfactory properties through physical crosslinking, and moreover, it is difficult to crosslink collagen so as to control the absorption rate *in vivo.*

In the case where the brain or various organs undergo a surgical operation due to diverse diseases or trauma and then the surgical wounds are closed, the incised dura mater, pericardium, pleura, peritoneum or serous membrane should be sutured again and closed. Due to such a suture, a shrunken portion may be formed, or the membrane may be partially excised, and thus the surgical wounds cannot be completely closed, and a defect may be created in the membrane.

When such a defect is allowed to remain, organs such as the brain, heart, lungs, and intestines may escape through the defect in the membrane, undesirably causing serious disorder or exposing the organs to the surrounding water or air, owing to which the surgical wounds do not heal.

Hence, a medical replacement membrane usable as a preservative for such a defect may be exemplified by lyophilized human dura mater collected from a dead body, a porous expanded polytetrafluoroethylene (EPTFE) film, polypropylene mesh, a Teflon sheet, a Dacron sheet, etc.

However, the use of human dura mater may entail a concern about epileptic seizures after surgery due to the adhesion of preserved human dura mater and brain parenchyma tissue. In particular, EPTFE is not degraded *in vivo* but may remain as a foreign material, thereby readily leading to infection. Furthermore, when it contacts the bio-tissue, tissue cells may generate apodyopsis. In this way, the existing membranes are known to cause many postoperative complications.

Accordingly, various materials for biomembranes, which possess biochemical properties, have properties suitable for suture treatment, and are able to retain their shapes for a predetermined period of time after application *in vivo,* are being developed.

Related techniques include Korean Patent Application Publication No. 10-2002-0036225 (Laid-open date: May 16, 2002, Title: Biomembrane for the protection and treatment of skin disease) and Korean Patent No. 10-1280722 (Registration Date: June 25, 2013, Title: Thin film multilocular structure made of collagen, member for tissue regeneration containing the same, and method for producing the same).

JP H06 166850 discloses a method for forming a practical transparent homogeneous thin film from a cocoon solution.

### [Disclosure]

### [Technical Problem]

Accordingly, an object of the present invention is to provide a method of manufacturing an artificial biomembrane using a silk matrix, wherein the manufacturing process is relatively simple, and thus the manufacturing cost may be reduced compared to when manufacturing typical artificial biomembranes. The artificial biomembrane may exhibit high cell culture capacity and biocompatibility, and have superior tensile strength and elongation, which are required on biomembranes.

The technical problem according to the present invention is not limited to the above objects, and other objects that are not described herein will be obviously understood by those having ordinary skill in the art from the following description.

### [Technical Solution]

In order to accomplish the above objects, the present invention provides a method of manufacturing an artificial biomembrane using a silk matrix, comprising a first step of inducing a silkworm to spin a planar silk matrix by moving a sheet on which the silkworm is placed, and a second step of subjecting the silk matrix having a cross-section with a first thickness to planar division into two or more silk matrix pieces having a predetermined shape with the first thickness.

The invention also provides a method of manufacturing an artificial biomembrane using a silk matrix, comprising a first step of inducing a silkworm to spin a planar silk matrix by moving a sheet on which the silkworm is placed, and a second step of subjecting the silk matrix having a cross-section with a first thickness to thickness division into two or more silk matrix portions having a second thickness, which is less than the first thickness.

Disclosed herein is also an artificial biomembrane made from a silk matrix, which is configured such that a silk matrix having a cross-section with a first thickness, produced from silkworms, is subjected to planar division into two or more silk matrix pieces having a predetermined shape with the first thickness.

Also disclosed herein is an artificial biomembrane made from a silk matrix, which is configured such that a silk matrix having a cross-section with a first thickness, produced from silkworms, is subjected to thickness division into two or more silk matrix portions having a second thickness, which is less than the first thickness.

Also disclosed herein is an artificial biomembrane using a silk matrix, which is configured such that a silk matrix having a cross-section with a first thickness, produced from silkworms, is subjected to planar division into two or more silk matrix pieces having a predetermined shape with the first thickness, and each of the silk matrix pieces having the first thickness is subjected to thickness division into silk matrix pieces having a second thickness, which is less than the first thickness.

### [Advantageous Effects]

According to the present invention, the method of manufacturing produces an artificial biomembrane which has superior tensile strength and elongation, which are required of biomembranes, and which can also manifest high cell culture capacity and biocompatibility. Furthermore, the manufacturing process thereof is simple, thereby reducing the manufacturing cost compared to when producing typical artificial biomembranes.

### [Description of Drawings]

FIG. 1 illustrates the production of a silk matrix for use in an artificial biomembrane from silkworms;
FIGS. 2A and 2B illustrate the results of visual observation of the morphology of the silk matrix at different thicknesses of 0.01 mm and 0.7 mm, respectively, used for the artificial biomembrane;
FIG. 3 illustrates the process of manufacturing the artificial biomembrane of Example 1 according to the present invention;
FIG. 4 illustrates the process of manufacturing the artificial biomembrane of Example 2 according to the present invention;
FIG. 5 illustrates the process of manufacturing the artificial biomembrane of Example 3 according to the present invention;
FIG. 6 illustrates the scanning electron microscopy (SEM) image of the silk matrix used for the artificial biomembrane; and
FIG. 7 illustrates the cell culture capacity of the artificial biomembrane.

### [Best Mode]

Unless otherwise stated, the meanings of the terms, descriptions, etc., disclosed in the present specification may be those that are typically used in the art to which the present invention belongs. Hereinafter, a detailed description will be given of the present invention.

A biomembrane refers to a membrane that constitutes a cell membrane or an organelle. That is, it functions as a boundary with the outside to prevent nucleic acids, proteins, and other biomaterials from being discharged to the outside, and is responsible for creating an independent environment to sustain life activity. Furthermore, a biomembrane is provided around the cells to protect the cells from the external environment, and plays a role as the passage for the transport of materials between the cytoplasm and the external environment.

The biomembrane has to have properties suitable for a suture process, and has to retain its shape for a predetermined period of time even after application *in vivo.* Also, the biomembrane is composed of a material that prevents adhesion to surgical wounds, entails no concern about infection, does not cause tissue degeneration, and promotes regeneration activity.

An artificial biomembrane is generally utilized as an artificial medical replacement membrane for use in an artificial neural tube, an artificial spinal cord, an artificial esophagus, artificial organs, artificial blood vessels, artificial valves, replaceable dura mater, artificial drum skins, etc.

However, the artificial biomembrane, which is currently available, is pretreated through physical crosslinking, etc. in order to ensure the above properties, but is problematic because it may become toxic *in vivo* upon long-term application, or it may remain as a foreign material *in vivo.* Furthermore, such an artificial biomembrane is made of expensive material because pretreatment and chemical treatment processes are performed in order to ensure the above properties, and thus the use thereof is difficult.

Therefore, in the present invention, the artificial biomembrane using a silk matrix is manufactured, the use of which is relatively simple, thus considerably reducing the production cost thereof compared to when manufacturing typical artificial biomembranes. Moreover, such an artificial biomembrane may exhibit superior cell culture capacity, biocompatibility, high tensile strength, and high elongation. Below is a detailed description thereof, made with reference to various examples.

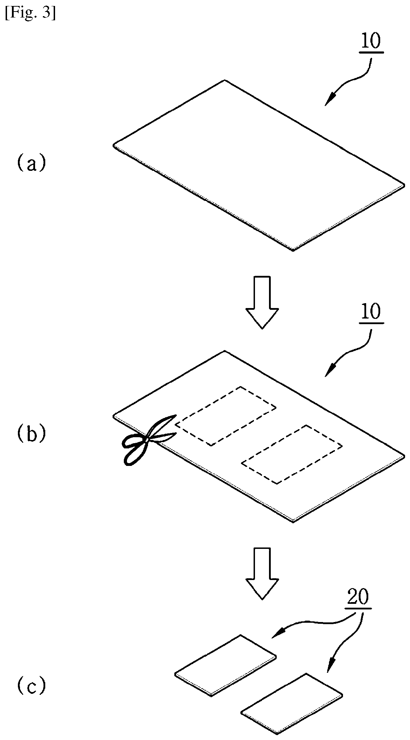

As illustrated in FIG. 3, the method of manufacturing an artificial biomembrane using a silk matrix of Example 1 according to the present invention is described below.

### 1. Preparation of silk matrix pieces having first thickness

A silk matrix having a cross-section with a first thickness, produced from silkworms, is subjected to planar division into two or more pieces having an appropriate shape, thus obtaining silk matrix pieces having the first thickness.

When silkworms become mature silkworms to protect themselves, they begin to transform into pupae by spinning cocoons. When a silkworm transforms into a pupa, it spins a cocoon to make a pupal casing having a round and elongated shape. The casing is oval-shaped, and shows various colors depending on the kind of silkworm, and both ends thereof are slightly pointed and are thick. However, in the course of building cocoons by the silkworms, when a sheet on which the silkworms are placed is moved so as to disturb the formation of normal cocoons by the silkworms, silkworms do not make cocoons. In the method of the invention, the silkworms are artificially induced to spin cocoons on the sheet by moving the sheet on which the silkworms are placed, whereby the cocoons are spun in the form of a planar sheet, yielding a planar silk matrix.

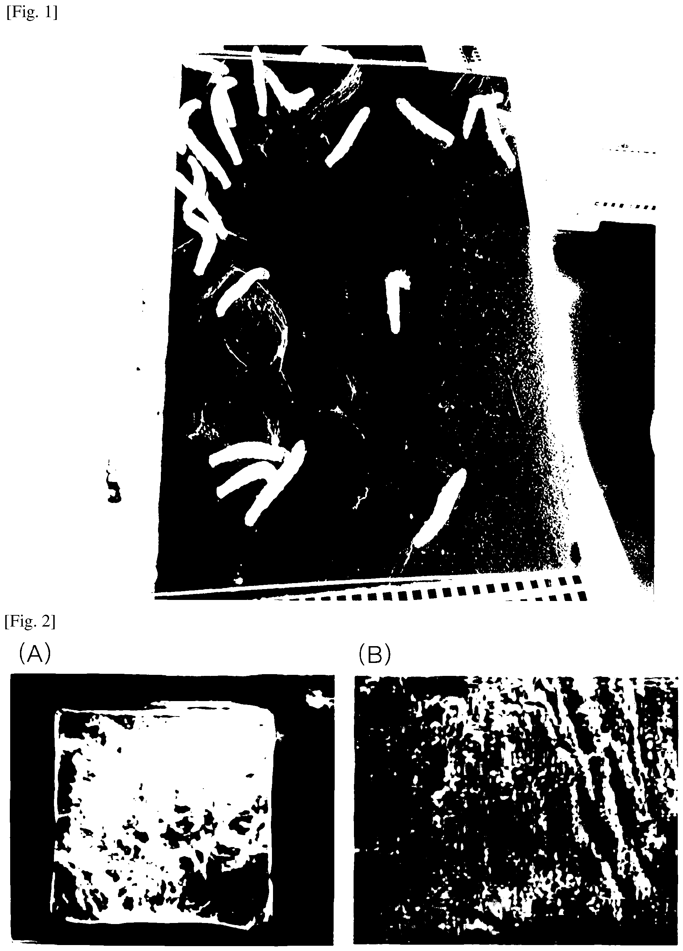

Based on the above features, as illustrated in FIG. 1 according to an embodiment of the present invention, silkworms are placed on a sheet, and the sheet is tilted at a slope (15 to 30°) to an extent that the silkworms are prevented from falling off the sheet, so that the silkworms are forced to move on the sheet and induced to spin cocoons, thus preparing a planar silk matrix (FIGS. 2A and 2B), having a cross-section with a first thickness, of interest in the present invention, which is then subjected to planar division into two or more pieces having an appropriate shape so as to be suitable for desired applications, yielding silk matrix pieces 20 having the first thickness, as illustrated in FIG. 3. Briefly, the silk matrix having a cross-section with a first thickness is subjected to planar division using a cutter such as a blade or scissors, thus forming the silk matrix pieces 20 having the first thickness and an appropriate shape.

As illustrated in FIG. 2A, the silk matrix pieces 20 having the first thickness and the appropriate shape, obtained by subjecting the silk matrix 10 having the first thickness to planar division, may be used unchanged because the silk matrix 10 having the first thickness is produced to a thickness suitable for use as an artificial biomembrane. Further, packing or sterile treatment and chemical treatment may be additionally performed, as necessary.

The sheet on which silkworms are placed is moved to form a planar silk matrix, the movement thereof may be carried out variously. The sheet may be moved in a manner of being rotated, repeatedly tilted upward and downward and/or leftward and rightward, or vibrated. Alternatively, the sheet may be moved through a combination of rotation and repeated tilting upward and downward and/or leftward and rightward. The number or rate of movement or tilting processes may be appropriately set in order to ensure that the silk matrix has the desired shape.

The shape of the sheet on which silkworms are placed is not limited. A circular shape or a rectangular shape may be applied. Alternatively, any shape may be applied so long as silkworms do not spin cocoons normally in response to the movement of the sheet.

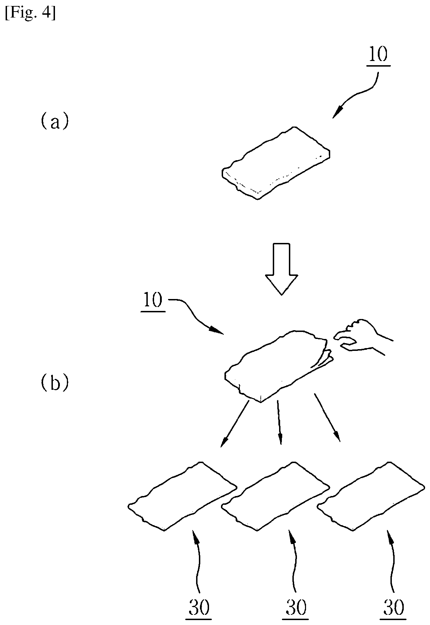

In another modification, the method of manufacturing an artificial biomembrane using a silk matrix of Example 2 according to the present invention, as illustrated in FIG. 4, is specified below.

### 1. Preparation of silk matrix portions having second thickness

A silk matrix having a cross-section with a first thickness, produced from silkworms, is subjected to thickness division into two or more portions having a second thickness, which is less than the first thickness.

The silk matrix 10 having the first thickness is produced in the method of the invention from silkworms in such a manner that silkworms spin cocoons while being forced to move on the sheet, as in the description of FIG. 3. The silk matrix 10 having the first thickness may be configured as illustrated in FIG. 2B such that yarns resulting from spinning cocoons from the silkworms are stacked in a height direction to thus form various thicknesses (multiple layers).

The first thickness of the silk matrix 10 may vary depending on the number of silkworms used to prepare the silk matrix and the period of time that the silkworms require to spin cocoons.

As shown in FIG. 4, when the silk matrix 10 having the first thickness is thick, it may be subjected to thickness division into two or more portions having a thickness suitable for use in an artificial biomembrane, thus forming silk matrix portions 30 having a second thickness. The second thickness is less than the first thickness owing to such thickness division.

The silk matrix portions 30 having the second thickness may be used unchanged as the artificial biomembrane. Alternatively, portions of the silk matrix portions 30 having the second thickness may be utilized by adjusting the size thereof so as to be suitable for some end use. Further, packing or sterile treatment and chemical treatment may be additionally performed, as necessary.

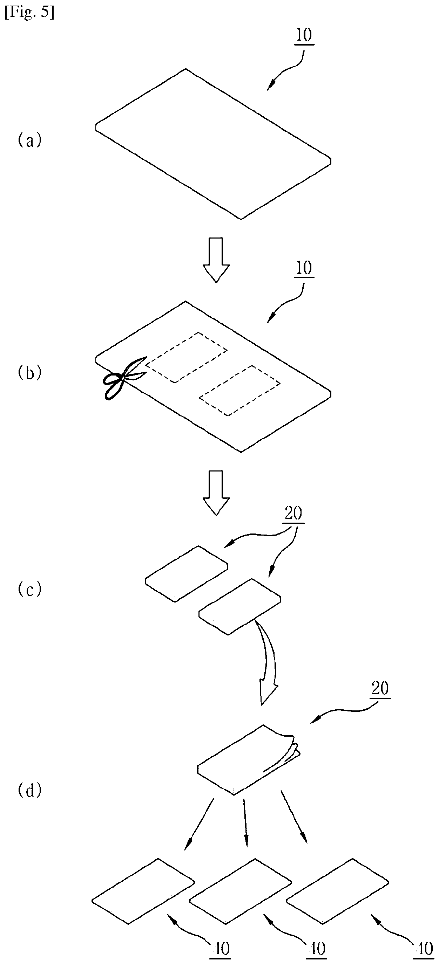

In a further modification, the method of manufacturing an artificial biomembrane using a silk matrix of Example 3 according to the present invention, as illustrated in FIG. 5, is specified below.

### 1. Preparation of silk matrix pieces having first thickness

A silk matrix having a cross-section with a first thickness, produced from silkworms in the method of the invention, is subjected to planar division into two or more pieces having an appropriate shape, thus forming silk matrix pieces 20 having the first thickness.

In this case, the silk matrix pieces 20 having the first thickness are formed in the same manner as in the first step of the description of FIG. 3.

When the first thickness of the silk matrix pieces 20 thus formed is suitable for use in an artificial biomembrane, such silk matrix pieces may be used without change. In the case where these silk matrix pieces are thick and difficult to use, they should be additionally subjected to the following step so as to have a thickness suitable for artificial biomembranes. The step is described below.

### 2. Preparation of silk matrix pieces (artificial biomembrane) having second thickness

Silk matrix pieces 40 having a second thickness, suitable for use as an artificial biomembrane, are prepared.

The silk matrix pieces 20 having the first thickness, obtained in the method of the invention, are configured as illustrated in FIG. 2B such that yarns resulting from spinning cocoons from the silkworms are stacked in a height direction to thus form various thicknesses (multiple layers).

The first thickness of the silk matrix pieces 20 may vary depending on the number of silkworms used to prepare the silk matrix and the period of time that the silkworms require to spin cocoons.

When the silk matrix pieces 20 having the first thickness are suitable for use in an artificial biomembrane, they may be used without change. As illustrated in FIG. 5, in the case where the silk matrix pieces are thick, each of the silk matrix pieces having the first thickness, obtained in the first step, is subjected to thickness division into two or more pieces having an appropriate thickness so as to serve as an artificial biomembrane, yielding silk matrix pieces 40 having a second thickness. As such, the second thickness is less than the first thickness owing to the thickness division.

The silk matrix pieces 40 having a second thickness may be used unchanged as an artificial biomembrane for any purpose. Further, packing or sterile treatment and chemical treatment may be additionally performed, as necessary.

A better understanding of the present invention may be obtained through the following examples and test examples, which are set forth to illustrate, but are not to be construed to limit the scope of the present invention.

### <Example 1> Manufacture of artificial biomembrane 1 of the invention

As illustrated in FIG. 1, 15 mature silkworms were placed on a sheet, and the sheet was gradually tilted at 20° with respect to the ground. After 1 hr, the sheet was gradually tilted again in the opposite direction. In this way, the silkworms spun cocoons while moving, yielding a silk matrix 10 in the conformation shown in FIG. 2A.

The above tilting process was performed for one day, equilibrium was maintained for two days, and the above tilting process was performed again, whereby a silk matrix was produced from the silkworms in about three days. The silk matrix had a thickness of about 0.01 mm.

As shown in FIG. 3, the silk matrix 10 was cut using scissors so as to undergo planar division in a rectangular shape, thus forming silk matrix pieces 20, which were then sterilized, thereby manufacturing an artificial biomembrane 1 according to the present invention.

### <Example 2> Manufacture of artificial biomembrane 2 of the invention

As illustrated in FIG. 1, 30 mature silkworms were placed on a sheet, and the sheet was gradually tilted at 20° with respect to the ground. After 1 hr, the sheet was gradually tilted again in the opposite direction. In this way, the silkworms spun cocoons while moving, yielding a silk matrix 10 in the conformation shown in FIG. 2B.

The above tilting process was performed for one day, equilibrium was maintained for two days, and the above tilting process was performed again, whereby a silk matrix was produced from the silkworms in about three days. The silk matrix had a thickness of about 0.7 mm.

As shown in FIG. 4, the silk matrix 10 having a thickness of 0.7 mm was peeled so as to undergo thickness division, thus forming silk matrix portions 30 having a thickness of 0.08 mm, which were then sterilized, thereby manufacturing an artificial biomembrane 2 according to the present invention.

### <Example 3> Manufacture of artificial biomembrane 3 of the invention

As illustrated in FIG. 1, 30 mature silkworms were placed on a sheet, and the sheet was gradually tilted at 20° with respect to the ground. After 1 hr, the sheet was gradually tilted again in the opposite direction. In this way, the silkworms spun cocoons while moving, yielding a silk matrix 10 in the conformation shown in FIG. 2B.

The above tilting process was performed for one day, equilibrium was maintained for two days, and the above tilting process was performed again, whereby a silk matrix was produced from the silkworms in about three days. The silk matrix had a thickness of about 0.7 mm.

The silk matrix was then cut using scissors so as to undergo planar division in a rectangular shape, thus forming silk matrix pieces 20.

As illustrated in FIG. 5, the silk matrix pieces 20 having a thickness of 0.7 mm were peeled so as to undergo thickness division, thus obtaining silk matrix pieces 40 having a thickness of 0.08 mm, which were then sterilized, yielding an artificial biomembrane 3 (silk matrix A) according to the present invention.

### <Example 4> Manufacture of artificial biomembrane 4 of the invention

An artificial biomembrane 4 (silk matrix B) according to the present invention was manufactured in the same manner as in Example 3, with the exception that silk matrix pieces 40 having a thickness of 0.2 mm were formed.

### <Example 5> Manufacture of artificial biomembrane 5 of the invention

An artificial biomembrane 5 (silk matrix C) according to the present invention was manufactured in the same manner as in Example 3, with the exception that silk matrix pieces 40 having a thickness of 0.4 mm were formed.

### <Test Example 1> Evaluation of morphology of silk matrix used for artificial biomembrane of the invention

### 1. Test Method

The morphology of the silk matrix was observed using SEM, as illustrated in FIGS. 2A and 2B.

### 2. Test Results

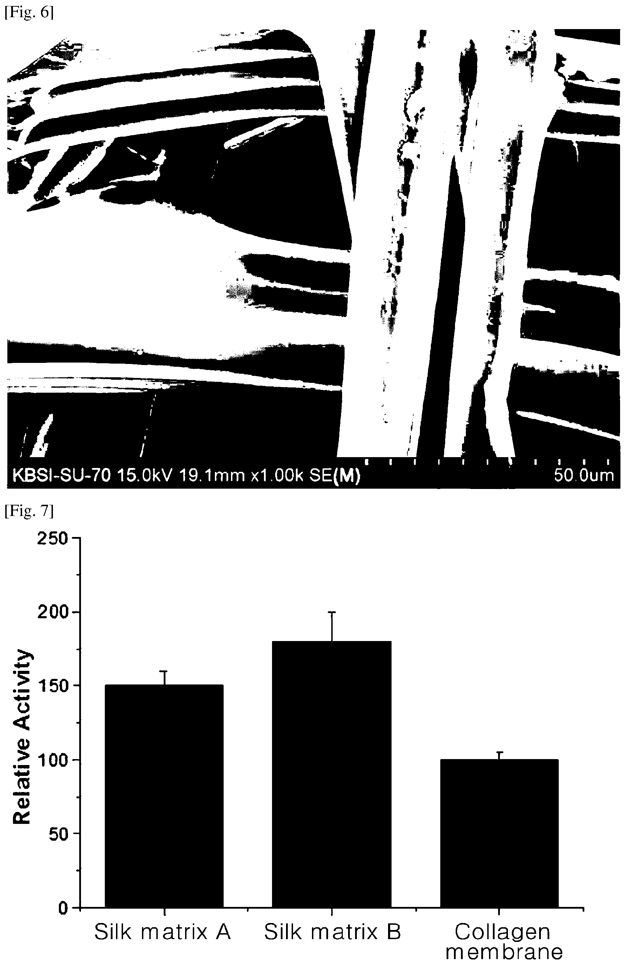

As illustrated in FIG. 6, the silk matrix had a smooth surface and thus a water-proof effect, and was not very sticky, and was thus suitable for use as a biomembrane. Further, the silk matrix had high porosity and thereby exhibited superior cell growth capacity and was thus suitable for a biomembrane that requires quick repair of a defect.

### <Test Example 2> Measurement of mechanical properties of artificial biomembrane using silk matrix according to the present invention

### 1. Test Method

In order to measure the mechanical properties of the artificial biomembrane using the silk matrix according to the present invention depending on the thickness thereof, tensile testing was performed using a universal testing machine (DAEYEONG, Korea). To this end, a dry sample and a wet sample resulting from immersion in saline for 1 hr were measured to determine the mechanical properties thereof. As a control, a commercially available collagen membrane was used. Analytical samples were manufactured to a size of 20 × 2.5 (width × length) mm, and the manufactured membrane was stretched at a gauge length of 20 mm and a rate of 10 mm/min. The results are shown in Table 1 below.

### 2. Test Results

**[Table 1]**

| Kind | Thick. (mm) | Max. Load (N) | | Tensile Strength (MPa) | | Elongation (%) | |
|---|---|---|---|---|---|---|---|
| | | Dry | Wet | Dry | Wet | Dry | Wet |
| Ex. 3 | 0.08 | 33 | 17 | 9 | 7 | 2.4 | 26.1 |
| Ex. 4 | 0.2 | 56 | 38 | 21 | 14 | 2.5 | 25.4 |
| Ex. 5 | 0.4 | 108 | 74 | 26 | 17 | 2.7 | 29.8 |
| collagen | 0.2 | 3.3 | 0.25 | 0.8 | 0.05 | 7.8 | 18.8 |

As is apparent from Table 1, the artificial biomembrane using the silk matrix according to the present invention exhibited variable tensile strength and elongation depending on the thickness thereof. The tensile strength and elongation were increased with an increase in the thickness of the membrane, and were also superior in a wet state, rather than a dry state. However, the tensile strength of the collagen membrane useful as a commercially available artificial biomembrane was decreased by a factor of 1/16 in a wet state. Therefore, the artificial biomembrane using the silk matrix, according to the present invention, maintained its properties for a long period of time compared to the collagen membrane in a wet state.

### <Test Example 3> Measurement of cell culture capacity of artificial biomembrane of the invention

### 1. Test Method

In order to evaluate the cell culture capacity of the artificial biomembrane, mouse-derived fibroblasts L929 were cultured on the artificial biomembrane under conditions of 37°C and 5% CO₂. The medium for cell culture was a DMEM medium (Dulbecco's Modified Eagles Medium-high glucose, WelGENE, Korea), containing 10% (v/v) FBS (fetal bovine serum, GIBCO), 100 U streptomycin, and 100 µg/ml penicillin (GIBCO). The cell culture capacity of the artificial biomembrane was measured in terms of relative activity using an MTT method.

The results are shown in FIG. 7.

### 2. Test Results

As illustrated in FIG. 7, the artificial biomembrane (Example 4) according to the present invention manifested higher cell growth capacity than the conventional collagen membrane.

That is, the artificial biomembrane according to the present invention can be confirmed to be suitable for use as a biomembrane requiring cell growth capacity.

## Claims

1. A method of manufacturing an artificial biomembrane using a silk matrix, comprising:
a first step of inducing a silkworm to spin a planar silk matrix by moving a sheet on which the silkworm is placed, and
a second step of subjecting the silk matrix having a cross-section with a first thickness to planar division into two or more silk matrix pieces having a predetermined shape with the first thickness.

2. A method of manufacturing an artificial biomembrane using a silk matrix, comprising:
a first step of inducing a silkworm to spin a planar silk matrix by moving a sheet on which the silkworm is placed, and
a second step of subjecting the silk matrix having a cross-section with a first thickness to thickness division into two or more silk matrix portions having a second thickness, which is less than the first thickness.

3. The method of claim 1, wherein the method further comprises:
a third step of subjecting each of the silk matrix pieces having the first thickness to thickness division into two or more silk matrix pieces having a second thickness, which is less than the first thickness.

4. The method of claim 1, further comprising a third step of packing the silk matrix pieces having the first thickness obtained in the second step.

5. The method of claim 2, further comprising a third step of packing the silk matrix portions having the second thickness obtained in the second step.

6. The method of claim 3, further comprising a fourth step of packing the silk matrix pieces having the second thickness obtained in the third step.

7. The method of any one of claims 1 to 6, further comprising performing at least one sterilization process, before or after each step.

## Patentansprüche

1. Verfahren zum Herstellen einer künstlichen Biomembran unter Verwendung einer Seidenmatrix, Folgendes beinhaltend:
einen ersten Schritt des Veranlassens einer Seidenraupe zum Spinnen einer planaren Seidenmatrix durch Bewegen eines Bogens, auf welchem die Seidenraupe platziert ist, und
einen zweiten Schritt des Unterwerfens der Seidenmatrix, welche einen Querschnitt mit einer ersten Dicke besitzt, unter eine planare Unterteilung in zwei oder mehr Seitenmatrixteile, welche eine vorbestimmte Form mit der ersten Dicke besitzen.

2. Verfahren zum Herstellen einer künstlichen Biomembran unter Verwendung einer Seidenmatrix, Folgendes beinhaltend:
einen ersten Schritt des Veranlassens einer Seidenraupe zum Spinnen einer planaren Seidenmatrix durch Bewegen eines Bogens, auf welchem die Seidenraupe platziert ist, und
einen zweiten Schritt des Unterwerfens der Seidenmatrix, welche einen Querschnitt mit einer ersten Dicke besitzt, unter eine Dickenunterteilung in zwei oder mehr Seidenmatrixabschnitte, welche eine zweite Dicke besitzen, welche geringer als die erste Dicke ist.

3. Verfahren nach Anspruch 1, bei welchem das Verfahren zudem Folgendes beinhaltet:
einen dritten Schritt des Unterwerfens eines jeden der Seidenmatrixteile, welche die erste Dicke besitzen, unter eine Dickenunterteilung in zwei oder mehr Seidenmatrixteile, welche eine zweite Dicke besitzen, welche geringer als die erste Dicke ist.

4. Verfahren nach Anspruch 1, zudem beinhaltend einen dritten Schritt des Packens der im zweiten Schritt erzielten Seidenmatrixteile, welche die erste Dicke besitzen.

5. Verfahren nach Anspruch 2, zudem beinhaltend einen dritten Schritt des Packens der im zweiten Schritt erzielten Seidenmatrixabschnitte, welche die zweite Dicke besitzen.

6. Verfahren nach Anspruch 3, zudem beinhaltend einen vierten Schritt des Packens der im dritten Schritt erzielten Seidenmatrixteile, welche die zweite Dicke besitzen.

7. Verfahren nach einen der Ansprüche 1 bis 6, zudem beinhaltend Durchführen mindestens eines Sterilisationsverfahrens, vor oder nach einem jeden Schritt.

## Revendications

1. Procédé de fabrication d'une biomembrane artificielle utilisant une matrice de soie, comprenant :
une première étape consistant à pousser un ver à soie à filer une matrice de soie plane en déplaçant une feuille sur laquelle est placé le ver à soie, et
une deuxième étape consistant à soumettre la matrice de soie présentant une coupe transversale avec une première épaisseur à une division plane en deux morceaux de matrice de soie ou plus, présentant une forme prédéterminée avec la première épaisseur.

2. Procédé de fabrication d'une biomembrane artificielle utilisant une matrice de soie, comprenant :
une première étape consistant à une première étape consistant à pousser un ver à soie à filer une matrice de soie plane en déplaçant une feuille sur laquelle est placé le ver à soie, et
une deuxième étape consistant à soumettre la matrice de soie présentant une coupe transversale avec une première épaisseur à une division d'épaisseur en deux parties de matrice de soie ou plus, présentant une seconde épaisseur, qui est inférieure à la première épaisseur.

3. Procédé selon la revendication 1, dans lequel le procédé comprend en outre :
une troisième étape consistant à soumettre chacun des morceaux de matrice de soie présentant la première épaisseur à une division d'épaisseur en deux morceaux de matrice de soie ou plus présentant une seconde épaisseur, qui est inférieure à la première épaisseur.

4. Procédé selon la revendication 1, comprenant en outre une troisième étape consistant à emballer les morceaux de matrice de soie présentant la première épaisseur obtenus dans la deuxième étape.

5. Procédé selon la revendication 2, comprenant en outre une troisième étape consistant à emballer les morceaux de matrice de soie présentant la seconde épaisseur obtenus dans la deuxième étape.

6. Procédé selon la revendication 3, comprenant en outre une quatrième étape consistant à emballer les morceaux de matrice de soie présentant la seconde épaisseur obtenus dans la troisième étape.

7. Procédé selon l'une quelconque des revendications 1 à 6, comprenant en outre la réalisation d'au moins un procédé de stérilisation, avant ou après chaque étape.
